# EUROPEAN PATENT APPLICATION

(11) **EP 2 095 780 A1**
(43) Date of publication of application: **02.09.2009**
(21) Application number: 07850301.8
(22) Date of filing: 07.12.2007
(51) Int. Cl.: A61B 17/3211

(54) **SURGICAL KNIFE, SURGICAL KNIFE BLADE AND METHOD OF PRODUCING THE SAME, AND SURGICAL KNIFE HANDLE**

(30) Priority: 08.12.2006 JP 2006332669
(71) Applicant: MANI Inc., Utsunomiya-shi, Tochigi 321-3231 (JP); Tokyo Metropolitan Industrial Technology Research Institute, Tokyo 115-8586 (JP)
(72) Inventor: SATAKE, Nozomi, Utsunomiya-shi Tochigi 321-3231 (JP); KAZAWA, Elito, Tokyo 115-8586 (JP)
(74) Representative: Prüfer & Partner GbR European Patent Attorneys
(86) International application number: PCT/JP2007/073723
(87) International publication number: WO 2008/069323

(57) **Abstract**

The blade of a surgical knife of the present invention is formed by subjecting a single-crystal silicon wafer in which the orientation of the polished surface is <110> or <100> to crystal anisotropic etching. The blade has an edge as a higher-order surface of crystal anisotropic etching, and the edge slopes at a sharp angle in relation to the polished surface. Therefore, the surgical knife of the present invention which is made of single-crystal silicon, has good sharpness, reduces variability in sharpness quality, has high productivity at low cost, and is practical.

## Description

### TECHNICAL FIELD

The present invention relates to a surgical knife used in ophthalmology, surgery, and other medical fields, and particularly relates to a blade for a surgical knife manufactured by crystal anisotropic etching of a single-crystal silicon wafer, a method of manufacturing the blade, a surgical knife that consists of the blade, and a handle for the surgical knife on which the blade is mounted.

### BACKGROUND OF THE INVENTION

A surgical knife for ophthalmology or surgery makes an incision in a body surface (skin, cornea, and the like), and is therefore required to have excellent sharpness. As long as the sharpness is excellent, the incision can be rectilinearly formed without causing more harm than necessary to the incision opening, healing can be hastened, and scar tissue is not left behind or can be made less noticeable. Also, with ophthalmic corneal surgery, post-surgical astigmatism can be avoided. For this reason, there is a need for the development of a surgical knife that has good sharpness and that can make a clean incision with little force.

Conventional surgical knives include metal-worked (stainless steel or the like) knives, and knives that have been polished of the crystal structure of a diamond. Metal knives are manufactured using methods that involve mechanical pressing, cutting, or lapping; methods that involve electrochemical machining (electroforming) or polishing; and methods that are combinations of the above. However, a metal knife has problems in that the lapping of the edge (sharpening edge tip) is limited, the sharpness is worse than a diamond knife, and the quality is not stable due to machining unstability. As used herein, the term "edge" refers to the end of a cutting edge.

With mechanical forming, machining precision and productivity are in an inverse proportional relationship. Specifically, machining unstability makes reduced sharpness quality attempting to produce larger quantities in a short period of time, and productivity is reduced with plenty of time.

On the other hand, a diamond knife can be manufactured for very good sharpness and stable quality by polishing along the crystal structure direction, but diamond knives have drawbacks such as expensiveness and productivity.

In view of the above, a surgical knife has been proposed in which single-crystal silicon is etched to manufacture the surgical knife (Patent Documents 1, 2 and 3). Etching includes dry etching and wet etching. Dry etching is a method of etching by reactive gas or ion beam, and wet etching is a method of etching by ions in a liquid. Isotropic etching and anisotropic etching can be classified by the direction in which the etching progresses. Since etching progresses at the same speed in all directions in isotropic etching, it is possible to use either wet etching or dry etching. Anisotropic etching includes wet etching in which the etching speed differs depending on the direction of the crystal structure, and dry etching that depends on the direction in which the ion beam is radiated. Since the etching direction of anisotropic wet etching depends on the crystal structure, this is named as a crystal anisotropic etching. A surgical knife in which the single-crystal silicon described in the Patent Document 1 is used is manufactured using a method in which a trench is formed in a wafer by cutting, the wafer is immersed in an isotropic etching solution, crystal material is uniformly removed, and a blade is obtained.

The blade disclosed in the Patent Document 2 uses a <100> single-crystal silicon, and has mutually parallel top and bottom surfaces composed of <100> planes and a cutting edge composed of <111> and <110> planes formed between the top surface and the bottom surface. The blade is formed by crystal anisotropic etching on a single-crystal silicon wafer. However, in the Patent Document 2, the edge is composed of only <111>, <110>, and other fundamental planes. The planes indicated by only 0's and 1's, such as <100>, <110>, <111>, and the like, are fundamental planes, and other planes are higher-order planes (<210>, <211>, <321>, and the like).

The blade described in the Patent Document 3 is one in which an edge composed of metal is milled using a focused ion beam (FIB) to form a sharp edge. This FIB is subjected to dry etching that depends on the beam direction.

Patent Document 1: National publication of the translated version of PCT application No. 2005-519703
Patent Document 2: US Patent US 7,059,054
Patent Document 3: European Patent EP 1092515A1

### DISCLOSURE OF THE INVENTION

### Problems the Invention Is Intended to Solve

However, the surgical knife described in the Patent Document 1 above is one in which machining and isotropic wet etching are used in combination, and therefore still has the problems of machining described above. In other words, there is a problem with the technique of the Patent Document 1 in that sharpness is poor and there is dispersion in the sharpness quality. Therefore, crystal structure is not used in the Patent Document 1, the shape and sharpness of the blade are limited by machining precision, and a practical knife shape and tip angle cannot be obtained.

In this manner, the method of forming a silicon blade using machining and isotropic etching is disadvantageous in terms of production in that the number of steps is increased and factors that cause unstable in quality are increased.

The blade described in the Patent Document 2 has the <100> plane as the top and bottom surfaces, and the inclined surfaces of the edge that slopes toward this plane are the <111> plane, the <110> plane, and other fundamental planes. Therefore, the angle that is formed by the surface and the inclined surface of the edge is greater than 54°. For this reason, the edge angle is large and good sharpness cannot be expected.

The blade described in the Patent Document 3 has an edge that is formed by FIB anisotropic etching (dry etching) with the aim of making the edge sharper, but the shape of the blade itself must be machined and dispersion in the shape is unavoidable.

In the conventional metal blade or diamond knife, a worker is injured when the worker touches the edge during the polishing step of the blade or other handling during manufacture, and the edge deforms and can no longer cut when contact is made with another object. It is also possible that the edge will no longer cut when contact is made during preparation or use in surgery, or at other times.

In the Patent Document 1, a substrate that has been lined with tape in advance is etched, whereby the blade can be prevented from moving and the edge or tip of the blade can be prevented from making contact with a hard object. However, there is a good possibility that the edge will be contacted by tweezers or another holding tool when the blade itself is removed from the tape and mounted in a handle.

A method of forming a blade is disclosed in the Patent Document 2, but the edge is liable to be damaged when contact is made with a hard object during the work of mounting the formed blade in a handle.

A pedestal for fixing the blade is disclosed in the Patent Document 3, but the edge is liable to be damaged when contact is made with a hard object during the work of packaging the edge.

Sharpness tests are ordinarily carried out via a service test by random sampling. Since sharpness is reduced when a blade is used once, such a test constitutes a destructive test. Therefore, the number of finished products is equal to the number of produced blades less the number of samples taken.

An object of the present invention is to provide a practical surgical knife, a handle for the surgical knife, and a blade for the surgical knife in which single-crystal silicon is used as the material, sharpness is good, dispersion in sharpness quality is reduced, costs are low, and productivity is high.

### Means for Solving the Problems

The surgical knife according to the present invention comprises: a blade in which a single-crystal silicon wafer in which the orientation of a polished plane is <110> or <100> is subjected to crystal anisotropic etching, whereby an inclined plane sloped in relation to the polished surface is formed; and an edge formed between the inclined plane and the polishing surface of the wafer.

In this surgical knife, the inclined plane constituting the edge is a higher-order plane, e.g., <322> and <311> planes, formed by crystal anisotropic etching when the orientation of the polished surface is <110>.

When the orientation of the polished surface is <100>, the inclined plane constituting the edge is a higher-order plane and a fundamental plane, e.g., <122> and <011>, formed by crystal anisotropic etching.

The blade for a surgical knife according to the present invention comprises: a blade in which a single-crystal silicon wafer in which the orientation of a polished surface is <110> or <100> is subjected to crystal anisotropic etching, whereby an inclined surface that slopes in relation to the polished surface is formed and an edge is formed between the inclined plane and the polished surface of wafer; a rim disposed in a position that is set at a distance from the blade at the periphery of the blade; and a rib for connecting the rim and the section of the blade in which the edge is not formed.

The method of manufacturing the blade for a surgical knife according to the present invention in a method comprising forming a mask pattern that includes a dummy section for inspection in addition to a product blade on the single-crystal silicon wafer; and etching the silicon wafer via crystal anisotropic etching using the mask pattern as a mask, whereby the surgical knife is manufactured.

The handle for a surgical knife of the present invention is one that is used for separating the blade for a surgical knife from the rib and forming a surgical knife in which the blade is mounted at a distal end, the handle having a handle main body; an open/close section which is disposed on the front end of the handle main body and in which a rear end section is rotatably supported on the handle main body so that a front end section opens and closes; and a projection which is disposed on a side margin of the open/close section and which disconnects the rib when the open/close section is closed.

### Effect of the Invention

In the present invention, single-crystal silicon, which is well-known as a semiconductor material, is used as a material. The shapes of a blade, a rim, a rib and a dummy blade are transferred to the polished surface of a single-crystal silicon wafer by photolithography, and since the single-crystal silicon wafer is processed with crystal anisotropic etching, ordinary semiconductor manufacturing techniques can be used in the manufacture of a surgical knife, and a surgical knife having high precision uniform sharpness can be obtained at low cost.

The edge can be sharpened to about several tens of times the scale of atomic bonding (lattice constant: 5.43 Å), i.e., 10 to 50 nm, by subjecting single-crystal silicon to crystal anisotropic etching. As a result, the contact surface area between the edge and the skin or another operative object is reduced, and sufficient force (pressure) to make an incision in the skin or the like can be obtained with little effort. For this reason, cutting can be performed in a simple manner without damaging the tip, and good incision can be obtained.

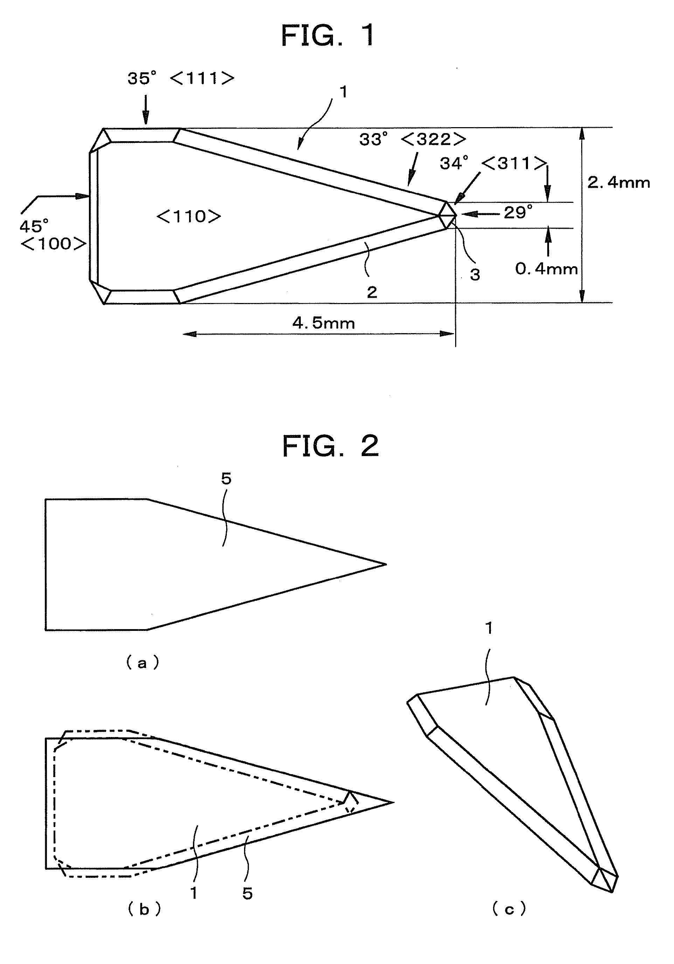

In the present invention according to claim 2, the orientation of the polished surface is <110>, and the inclined surface constituting the edge is, e.g., <322> and <311>, as shown in FIG. 1. Therefore, the angle formed by the <311> plane of the distal end of the blade and the back surface of the blade (polished surface) is 34°, the angle formed by the <322> plane of the side margin of the blade and the back surface of the blade is 33°, and a very sharp tip angle can be obtained, as shown in FIG. 1.

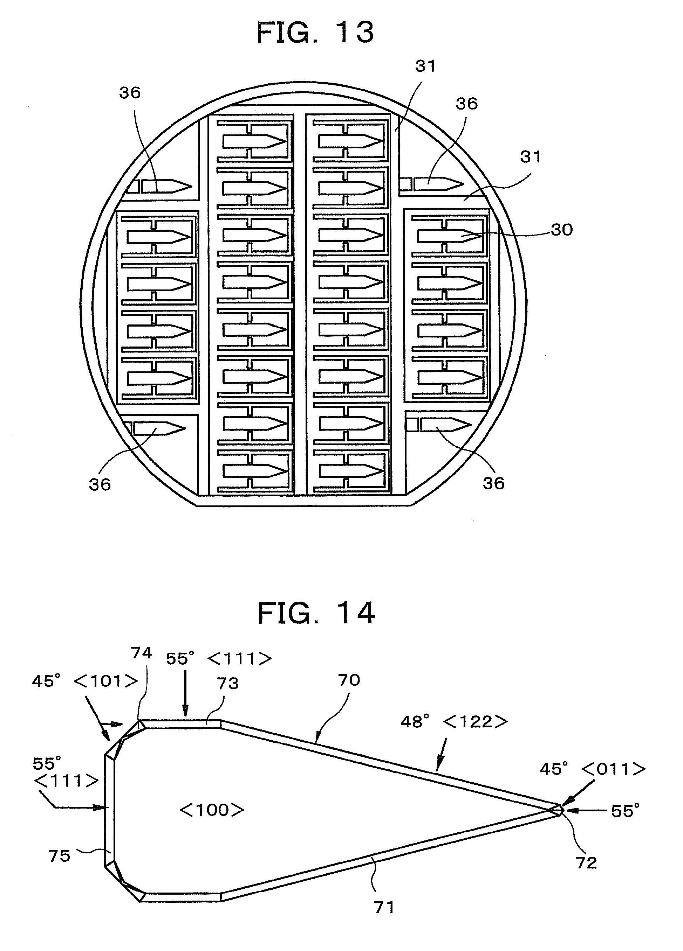

In the present invention according to claim 3, the orientation of the polished surface is <100>, and the inclined surface constituting the edge is, e.g., <122> and <011>, as shown in FIG. 14. Therefore, the angle formed by the <011> plane of the distal end of the blade and the back surface of the blade (polished surface) is 45°, the angle formed by the <122> plane of the side margin of the blade and the back surface of the blade is 48°, and a sharp tip angle can be obtained that is less than that of the blade described in the Patent Document 2, i.e., 54°, as shown in FIG. 14.

In the present invention according to claim 4 or 5, a mask pattern for blades and optional dummy blades is formed, for example, on a silicon wafer; a mask pattern for a rim is disposed on the periphery of the blades; and a mask pattern for ribs that connect the rims and the blades is formed, whereby a pattern of the blades, the rims, and the ribs can be formed on a silicon wafer using photolithography, which is well-known in semiconductor manufacturing technology. A blade is inserted into the opening of the open/close section using the handle described in the sixth aspect of the present invention in a state in which the open/close section is open, and the open/close section is closed, whereby the open/close section holds the blade, and the projection cleaves and disconnects the rib. Therefore, the blade is separated from the rib by a very simple operation, and the blade can be mounted on the distal end of the handle. Also, a worker is not required to grasp or hold the blade when the blade is mounted in the handle. Therefore, degradation of the sharpness of the blade that occurs when a worker's finger touches the edge of the blade can be prevented because the worker does not make contact with the blade. Fingers can also be prevented from being injured by the edge of the blades.

### BRIEF DESCRIPTION OF THE DRAWINGS

[FIG. 1]
   FIG. 1 is a top view showing the blade 1 of an embodiment of the present invention, in which single-crystal silicon having an orientation of <110> is used;
[FIG. 2]
   FIG. 2(a) shows a mask pattern 5, FIG. 2(b) shows the relationship between the mask pattern and the blade, and FIG. 2(c) shows a perspective view of the blade;
[FIG. 3]

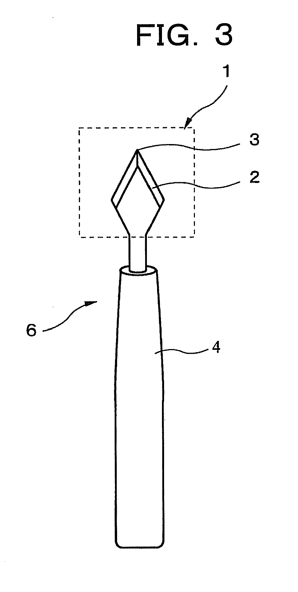

   FIG. 3 is a diagram showing a surgical knife;
[FIG. 4]

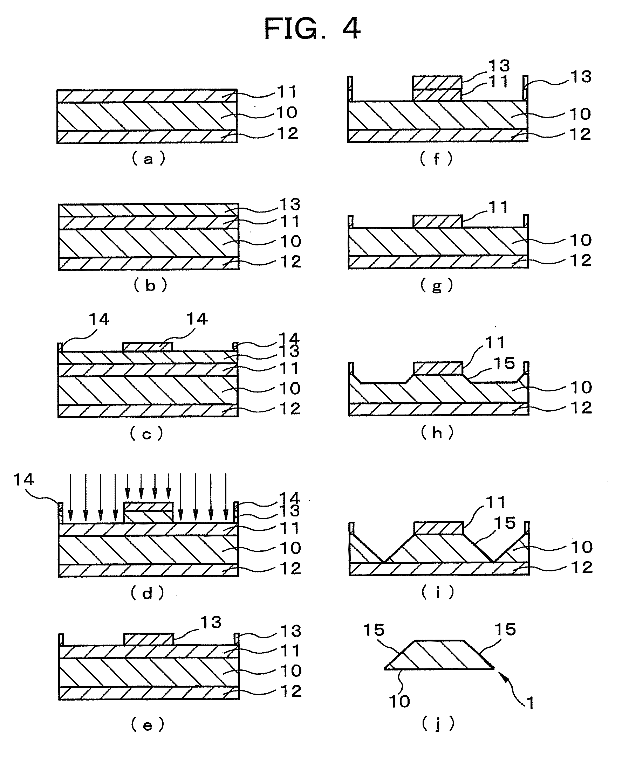

   FIG. 4 is a cross-sectional view showing, as a sequence of steps, the method of manufacturing a blade;
[FIG. 5]

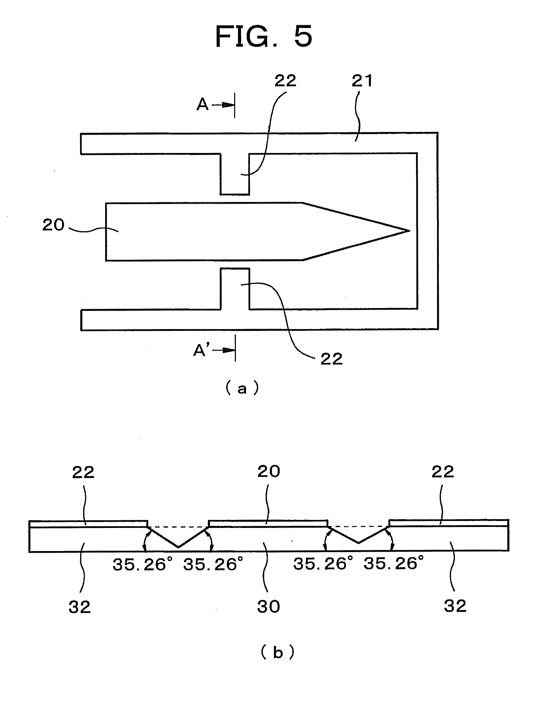

   FIG. 5(a) shows a mask pattern of a rimmed blade, and FIG. 5(b) is a cross-sectional view showing the mask pattern and the result of etching;
[FIG. 6]

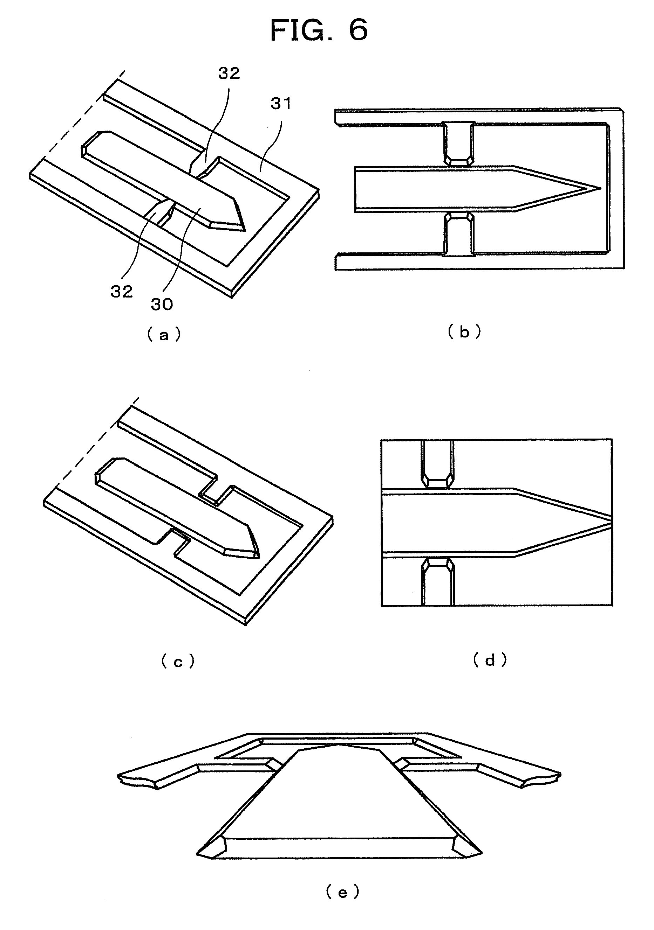

   FIG. 6(a) is a perspective view of the rimmed blade, FIG. 6(b) is a top view, FIG. 6(c) is a perspective view, FIG. 6(d) is an enlarged view showing the connection points between the rim and the blade, and FIG. 6(e) is a perspective view as seen from the rear of the blade;
[FIG. 7]

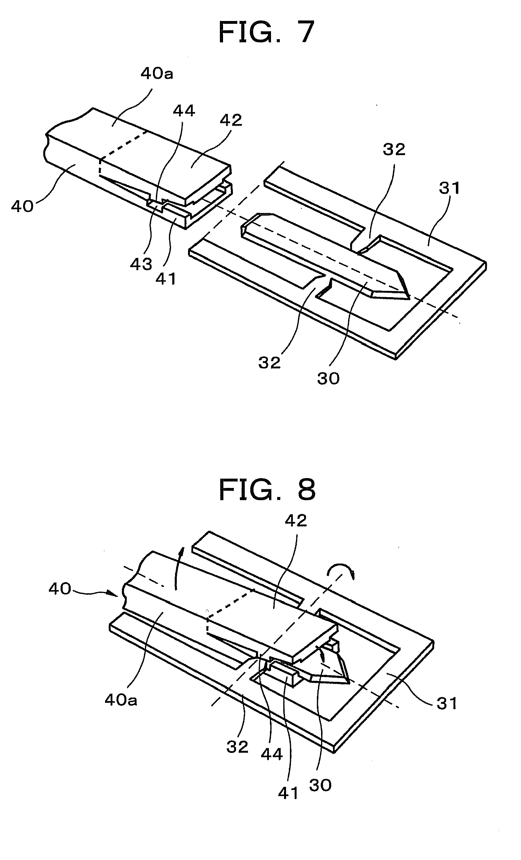

   FIG. 7 is a perspective view showing the handle of an embodiment of the present invention;
[FIG. 8]
   FIG. 8 is a perspective view showing the method of mounting a blade in a handle;
[FIG. 9]

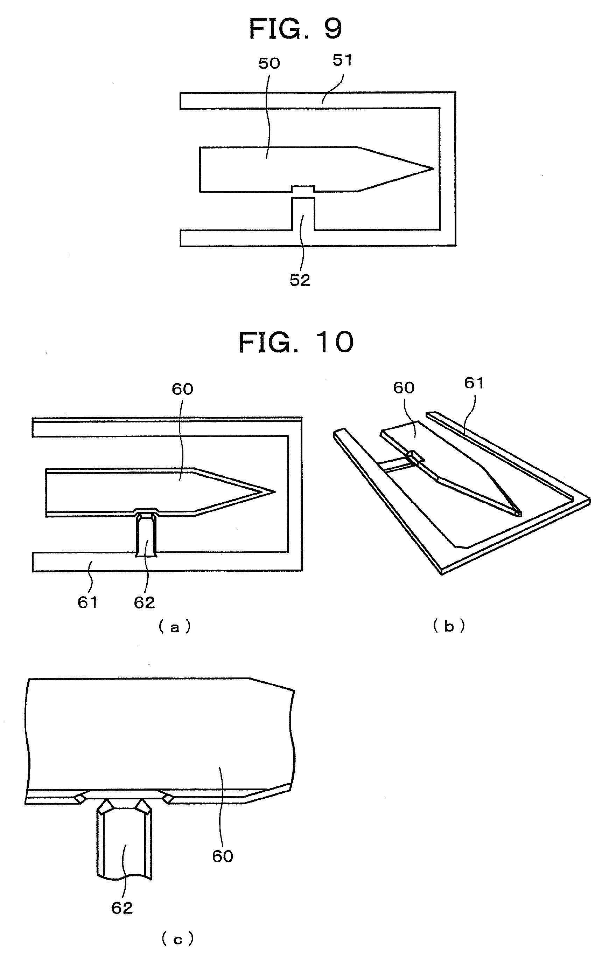

   FIG. 9 is a top view showing the mask pattern of a one-sided rib in which a rib is provided to one side of the blade;
[FIG. 10]
   FIG. 10(a) is a top view showing a rimmed blade for the case of a one-sided rib, FIG. 10(b) is a perspective view, and FIG. 10(c) is an enlarged top view of the connection part between the rib and the blade;
[FIG. 11]

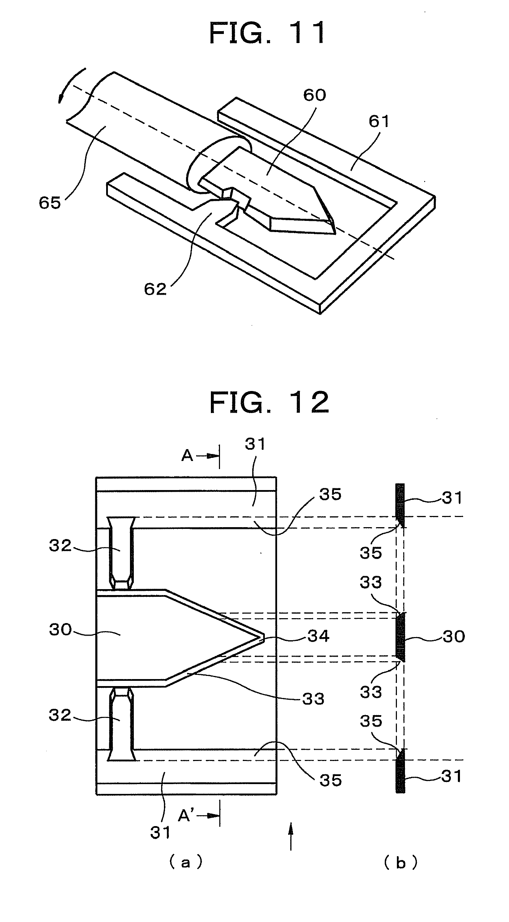

   FIG. 11 is a perspective view showing a rimmed blade in which a handle has been mounted in advance;
[FIG. 12]
   FIG. 12(a) is a top view showing a blade as well as a rimmed blade provided with a disposable blade, and FIG. 12(b) is a cross-sectional view;
[FIG. 13]
   FIG. 13 is a top view showing rimmed blades provided with disposable blades in the same manner;
[FIG. 14]
   FIG. 14 is a top view showing a blade of another embodiment of the present invention in which single-crystal silicon having an orientation of <100> is used;
[FIG. 15]

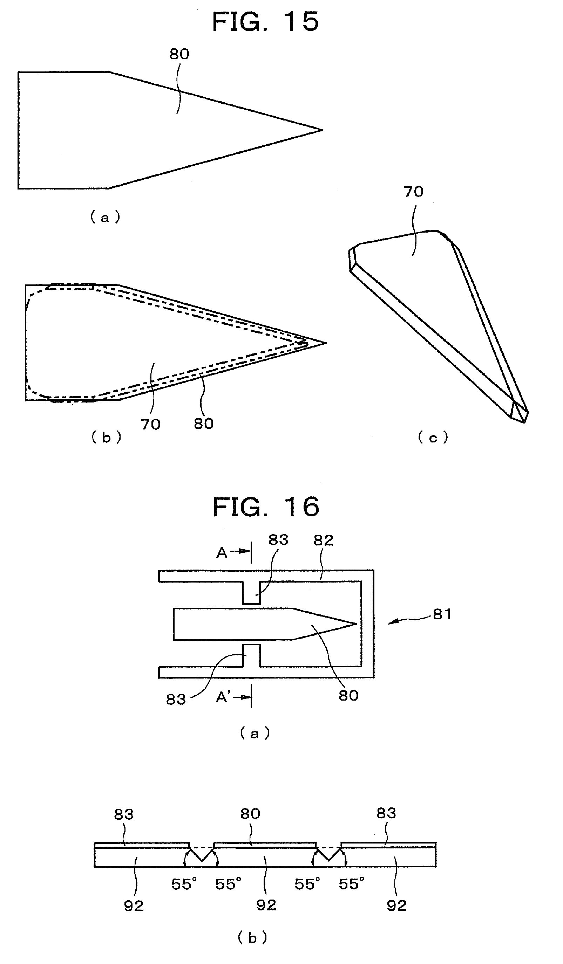

   FIG. 15(a) is a top view of a mask pattern, FIG. 15(b) is a view showing the relationship between the mask pattern and the blade, and FIG. 15(c) is a perspective view of the blade;
[FIG. 16]
   FIG. 16(a) is a mask pattern of a rimmed blade, and FIG. 16(b) is a cross-sectional view showing the mask pattern and the etching result; and
[FIG. 17]

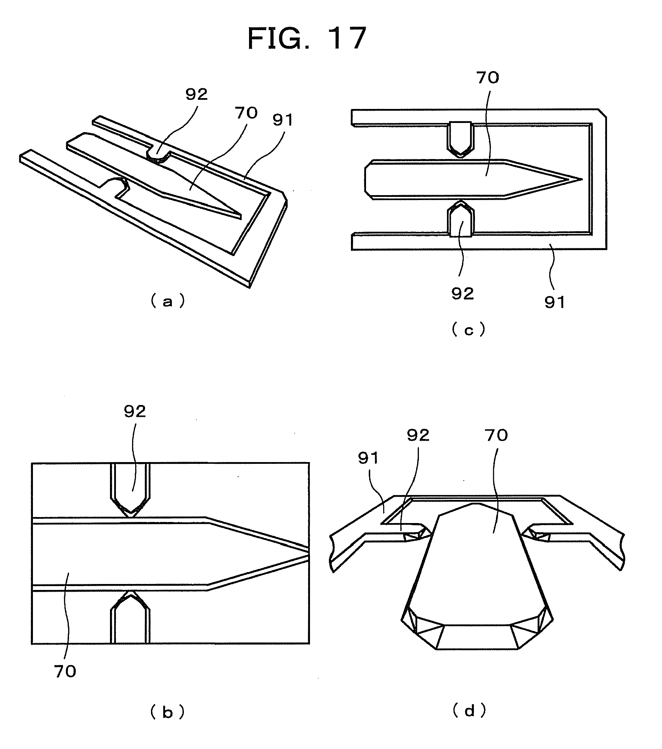

   FIG. 17(a) is a perspective view showing a rimmed blade, FIG. 17(n) is an enlarged view of the connection parts of the blade and rib, FIG. 17(c) is a top view of a rimmed blade, and FIG. 17(d) is a perspective view as seen from the rear of the blade.

### Description of Reference Numerals

- 1: blade
- 2: edge
- 3: tip
- 4: handle
- 5: mask pattern
- 6: knife
- 10: single-crystal silicon wafer
- 11: oxide layer
- 12: oxide layer
- 13: resist
- 14: mask pattern
- 15: inclined surface
- 20,: 21, 22, 82, 83 section
- 30: blade
- 31: rim
- 32: rib
- 33: edge
- 34: tip
- 35: dummy blade
- 36: dummy blade
- 40: handle
- 41: fixed section
- 42: open/close section
- 43: concave section
- 44: projection
- 50: section
- 51: section
- 52: section
- 60: blade
- 61: rim
- 62: rib
- 65: handle
- 70: blade
- 71: edge
- 72: tip
- 74: corner section
- 80,: 81 mask pattern
- 92: rib
- 40a: main body

### DESCRIPTION OF THE PREFERRED EMBODIMENTS

The present invention is described in detail below with reference to the attached diagrams. FIG. 1 is a top view that shows a blade 1 of the first embodiment of the present invention. In the blade 1, polished front and back surfaces on a single-crystal silicon wafer have the <110> orientation. A mask pattern 5 shown in FIG. 2(a) is formed on the wafer and is subjected to crystal anisotropic etching to obtain the blade 1 shown in the perspective view of FIG. 2(c). The silicon is eroded to below the edge section of the mask pattern 5 by crystal anisotropic etching, the side surface of the blade 1 becomes sloped, and the distal end of the blade 1 slightly recedes, as shown in FIG. 2(b), which is superimposed diagram of the blade 1 and the mask pattern 5.

Such a blade 1 is mounted at the distal end of a handle 4, then a surgical knife 6 is assembled, as shown in FIG. 3. The term "blade 1" refers to the section of the cutting edge that is mounted at the distal end of the handle 4, the distal end of the blade 1 is a tip 3, and the inclined surface of the side margin of the blade 1 is the edge that makes an incision in an object.

FIGS. 4(a) to 4(j) are cross-sectional views of a wafer showing, as a sequence of steps, a method for obtaining the blade 1 shown in FIG. 1 from a single-crystal silicon wafer. First, oxide layers 11 and 12 are formed on the polished front and back surfaces of a single-crystal silicon wafer 10, as shown in FIG. 4(a). A resist 13 is applied by spin coating in a thickness of about 1 µm to the oxide layer 11 on the surface of the single-crystal silicon wafer 10, as shown in FIG. 4(b).

Next, a hard mask (or pattern film) 14 in which a blade pattern has been formed in advance is superimposed on the resist 13, as shown in FIG. 4(c). The entire surface is exposed to UV rays, as shown in FIG. 4(d). Next, the hard mask (or pattern film) 14 is removed and the exposed wafer is developed and washed, whereupon the resist 13 of the portions covered by the mask pattern of the hard mask (or pattern film) 14 and unexposed to UV rays is left behind, and the resist 13 of the exposed portions is removed. The pattern of the resist 13 is formed when the hard mask (or pattern film) 14 is thereafter removed, as shown in FIG. 4(e).

Next, the oxide layer 11 of the portion not covered by the mask of the resist 13 is removed when the resist 13 is used as a mask, and is isotropically etched using buffered hydrofluoric acid or another hydrofluoric acid, as shown in FIG. 4(f). The pattern of the oxide layer 11 is formed on the surface of the single-crystal silicon wafer 10 when the resist 13 is removed, as shown in FIG. 4(g). The pattern of the oxide layer 11 is the mask pattern 5 shown in FIG. 2(a).

In this state, the single-crystal silicon wafer 10 is subjected to crystal anisotropic etching, as shown in FIGS. 4(g) to 4(i). The single-crystal silicon wafer 10 is immersed in etching fluid controlled for concentration and temperature for the crystal anisotropic etching, removing the single-crystal silicon wafer 10 from immersion after a prescribed length of time has elapsed, and washing the wafer. In this crystal anisotropic etching, etching is performed from the portions that are not covered by the oxide layer 11 on the single-crystal silicon wafer 10, revealing the <111> plane along the mask pattern and a higher-order plane in which etching progresses over time. In the peripheral portions of the oxide layer 11, an inclined surface 15 is thereby formed that slopes in relation to the front and back surfaces of the single-crystal silicon wafer 10, and etching progresses. Ultimately, the inclined surface 15 reaches the oxide layer 12, and the portion below the oxide layer 11 of the single-crystal silicon wafer 10 is separated from the adjacent portion. The oxide layers 11 and 12 are thereafter removed and the wafer is washed, as shown in FIG. 4(j).

The blade 1 shown in FIG. 1 is thereby obtained. The relationship at this time between the mask pattern (oxide layer 11) and the blade 1 thus formed is shown in FIG. 2B. Specifically, the blade 1 has front and back surfaces in which the orientation of the polished surface is <110>, the tip 3 of the distal end of the blade 1 is formed so that two <311> planes intersect at the center line of the blade 1, the width of the blade 1 increases from the tip 3 toward the rear, and an edge 2 is formed at the peripheral section of the widening section, as shown in FIG. 1. The side margin of the rear section of the blade 1 has an inclined surface formed on the rear end section as well. The inclined surface of the edge 2 is a <322> plane, the side margin of the rear section is a <111> plane, and the rear end section is a <100> plane.

In this manner, inclined surfaces having a specific orientation are formed by crystal anisotropic etching so that a <311> plane forms the tip 3, a <322> plane forms the edge 2, a <111> plane forms the rear section side margin, and a <100> plane forms the rear end section in the peripheral section of the blade 1 in which the front and back surfaces are a <110> plane. In this case, the <311> plane of the tip 3 forms an angle of 34° in relation to the front and back surfaces, and the <322> plane of the edge 2 forms an angle of 33° in relation to the front and back surfaces. The angle of the tip 3 in the center line direction of the blade in relation to the front and back surfaces is 29°. Accordingly, the angle formed by the section that forms the cutting edge in the blade 1 is very small, and a sharp surgical knife can be obtained. The angle formed by the <111> plane of the rear section side margin is 35°, and the angle formed by the <100> plane of the rear end section is 45°.

Such planes can be formed by a process in which a mask pattern (e.g., the acute angle of the distal end is 30°, and the rear section side margin is parallel to the <100> plane) such as that shown in FIG. 2 is formed in combination with the shape of the target blade, crystal anisotropic etching is performed using, e.g., KOH aqueous solution as the etching solution, and the etching time is controlled to reveal the <311> plane, the <322> plane, and other higher-order planes. An example of the dimensions of the resulting blade 1 is a width of 2.4 mm, a width of 0.4 mm for the two <311> planes, and a length of 4.5 mm in the blade center line direction of the edge 2 in a case in which the thickness of the wafer is set to 150 µm.

The approximate etching time is obtained by dividing the thickness of the wafer (e.g., 150 µm) by the etching rate of the <110> plane. The etching rate of the <110> plane is 10.4 µm/hour for the case in which KOH is the etching solution, the mass concentration is 25wt%, and the temperature is 40°C. The time must be strictly controlled because the edge is formed from a higher-order plane.

In the present embodiment, the higher-order planes constituting the inclined surfaces of the edge and tip formed by crystal anisotropic etching are <322> and <311>. An example of the etching conditions for forming the surfaces is described below.

The etching times are shown in TABLE 1 below for the case in which 20wt% KOH aqueous solution is used.

**[TABLE 1]**

| | Wafer orientation | Etching rate µm/hour | Etching time | |
|---|---|---|---|---|
| | | | Wafer thickness = 150 µm | Wafer thickness = 200 µm |
| T = 25°C | <100> | 2.865 | 53 hours | 70 hours |
| | <110> | 3.342 | 35 hours | 60 hours |
| T = 50°C | <100> | 17.178 | 8.8 hours | 11.7 hours |
| | <110> | 20.377 | 7.4 hours | 9.9 hours |

However, <322>, <311>, and other higher-order planes are often formed in close resemblance to the shape of the mask pattern. When a mask pattern in which the angle of the distal end of the knife is 30° is used, the edge 2 is between the <433> plane and the <322> plane in FIG. 1, and the edge angle is 31 to 32°. This is expressed as <322> in FIG. 1. A sharp edge can be obtained by using higher-order planes because the angle of the edge is 35.26° when the <111> plane is used in a <110> wafer.

The edge is a <21(-1)> plane and the edge angle is 30° when the angle of the distal end of the knife of the mask pattern is 70°.

In this manner, the orientation can be varied by the angle of the mask pattern even if the etching conditions are the same. Accordingly, the higher-order planes are not limited to <311> and <322> when a <110> wafer is used, and various higher-order planes can be used as the edge or tip.

As described above, a surgical knife having a very sharp edge can be obtained by using a prescribed mask pattern to perform crystal anisotropic etching on a single-crystal silicon wafer having a <110> orientation. Also, ordinary semiconductor manufacturing techniques can be used in the formation steps, resulting in ease of manufacture, low costs, and low dispersion in the sharpness quality.

The second embodiment of the present invention will be described next. FIG. 5(a) is a top view showing the mask pattern of the present invention, and FIG. 5(b) is a cross-sectional view along the line A-A of FIG. 5(a). FIG. 6(a) shows the shape of the resulting blade, FIG. 6(b) is a top view, FIG. 6(c) is a perspective view, FIG. 6(d) is an enlarged view showing the connection points between the rim and the blade, and FIG. 6(e) is a perspective view as seen from the rear of the blade. The mask pattern is composed of a section 20 that corresponds to a blade 30, a section 21 that corresponds to a rim 31 (not shown in FIG. 5, see FIG. 6), and a section 22 that corresponds to a rib 32, as shown in FIG. 5. The blade section 20 and the rib section 22 are set apart at a suitable distance. When such a mask pattern (sections 20, 21, and 22) is used and a single-crystal silicon wafer is subjected to crystal anisotropic etching, a blade 30, a rib 31, and a rim 32 can be obtained having a shape such as that shown in FIG. 6. In such a case, the side surface of the blade 30 and the rib 32 are inclined surfaces that slope in the manner shown in FIG. 5(b), and the blade 30 and rib 32 are connected by a section having a very slight thickness. The blade 30 shown in FIG. 6 is supported by a rib 32 and is disposed in a position in which the tip and edge of the blade 30 are surrounded by the rim 31. Therefore, the worker can be prevented from inadvertently making contact with the tip and edge of the blade 30 during handling of the blade 30 and becoming hurt, the tip and edge of the blade can be prevented from making contact with a hard object, and the tip and edge can be prevented from being damaged and degraded. Accordingly, the handling of the blade 30 is made very simple by providing a rim 31 and rib 32. In this manner, the blade 30 is supported by the rib 32, whereby the blade can be easily mounted in a handle as described below.

FIG. 7 is a perspective view showing the handle 40 for a surgical knife according to an embodiment of the present invention. FIG. 8 is a perspective view showing the method of mounting the blade. A worker does not make contact with the blade 30, and the handle 40 shown in FIG. 7 is mounted onto the blade 30, which is formed by subjecting the wafer to crystal anisotropic etching so that the rim 31 and rib 32 are connected by a section having a slight thickness, as shown in FIG. 6. The handle 40 has a handle main body 40a that the worker grips, and the distal end section of the handle main body 40a is provided with a fixed section 41 that constitutes a lower half section of the handle main body 40a, and an open/close section 42 that together with the fixed section 41 constitutes the distal end section of the handle main body 40a and that is rotatably supported on the handle main body 40a. A projection 44 is disposed at the two side peripheries of the open/close section 42, and a concave section 43 into which the projection 44 is fitted is disposed in a position that conforms to the projection 44 on the two side peripheries of the fixed section 41. At least the portion of the handle main body 40a disposed toward the distal end section is hollow, and when the fixed section 41 and open/close section 42 are mutually superimposed, a space is formed in the interior in a state in which the side walls make contact with each other. The distal end periphery of the fixed section 41 is U-shaped, and the blade 30 is held between the open/close section 42 and the fixed section 41 when the open/close section 42 is closed in a state in which the blade 30 is superimposed on the inside bottom surface of the fixed section 41.

In view of the above, the handle 40 is brought close to the blade 30 in a state in which the open/close section 42 is open, and the handle 40 is positioned with respect to the crystal anisotropically etched blade 30 so that the concave section 43 of the fixed section 41 is aligned with the connection portion of the rib 32 and the blade 30, as shown in FIG. 8. When the open/close section 42 is closed so that the open/close section 42 is superimposed on the fixed section 41, the projection 44 of the open/close section 42 is fitted into the concave section 43, and the connection portion between the rib 32 and blade 30 is severed thereby. The projection 44 of the open/close section 42 is fitted and simultaneously interlocked with the concave section 43 of the fixed section 41, and the blade 30 thus separated is thereby held between the open/close section 42 and fixed section 41 without the open/close section 42 opening. In this manner, the blade 30 is mounted in the handle 40 without the worker making contact with the blade 30. Therefore, sebum can be prevented from being deposited on the blade and degrading the sharpness of the blade.

Described next is a blade for a surgical knife that is connected by a rib only on the side margin of one side of the blade. FIG. 9 is a top view showing a mask pattern of the blade. The mask pattern is composed of a section 50 that corresponds to a blade, a rim section 51 that extends in the lengthwise and width directions of the blade section 50, and a rib section 52 that extends from the rim section 51 toward the blade section 50, as shown in FIG. 9. In the present embodiment, the rib section 52 is disposed only in the vicinity of the side margin of one side of the blade section 50, and the rib section 52 is patterned and formed so as to be slightly set at a distance from the blade section 50.

When single-crystal silicon is subjected to crystal anisotropic etching using the mask pattern shown in FIG. 9, a blade 60, rim 61, and rib 62 are obtained having the shapes shown in FIGS. 10(a), 10(b), and 10(c). The blade 60 is connected to a single rib 62 on one side margin.

FIG. 11 is a perspective view showing a rimmed blade on which a handle 65 has been mounted in advance. In contrast to mounting a blade when a surgical knife is to be used in the manner shown by the handle 40 of FIG. 8, this is an example of a product in which a handle has been mounted in advance. Examples of a method of fixing the blade 60 in the handle 65 include the use of an adhesive that is filled and set in the gap between the handle and the blade, and the use of welding in which a thermoplastic material is heated using the handle. In this manner, the blade 60 can be easily separated from the rib 62 by rotating the handle 65 about the center axis because the handle 65 has been mounted in advance on the blade 60.

Another embodiment of the present invention will be described next. FIG. 12(a) is a top view showing a rimmed blade provided with a dummy blade (disposable blade) that is used for checking sharpness, and FIG. 12(b) is a cross-sectional view along the line A-A. An inclined surface having a prescribed sharp angle is formed by crystal anisotropic etching at the edge 33 and the tip 34 of the blade 30, and an inclined surface is also formed at the same time on the side margin of the rim 31. The inclined surface formed on the side margin of the rim 31 is one that is sloped at the same cross-sectional angle as the inclined surface of the edge 33 of the blade 30, and is the dummy blade 35 formed on the rim 31. In view of the above, sharpness can be confirmed using the dummy blade 35 after the blade 30 has been demounted from the rimmed blade. The quality in the manufacturing step that includes crystal anisotropic etching can be checked/tested without wasting a product blade 30, and this checking/testing can be used in place of product shipment inspection. Alternatively, sharpness can be confirmed using the rim that is removed when blades are to be used.

FIG. 13 is a top view showing a modified example of the present embodiment. In the modified example, a dummy blade 36 having the same shape as the distal end of the product blade is formed outside of the area in which the product blade 30 are formed, and sharpness can be confirmed/tested using the dummy blade 36. In the modified example, the dummy blade 36 is mounted in a handle and assembled into a surgical knife, and the sharpness can be confirmed/tested using the surgical knife, whereby confirmation that is more proximate to actual conditions can be carried out.

Another embodiment of the present invention will be described next. The present embodiment is a case in which a blade is made of single-crystal silicon in which the polished surfaces of the front and back surfaces are <100> planes. FIG. 14 is a top view showing a blade 70 of a surgical knife of the present embodiment. FIG. 15(a) is a top view of a mask pattern 80 for manufacturing the blade, FIG. 15(b) is a view showing the relationship between the blade 70 and the mask pattern 80, and FIG. 15(c) is a perspective view of the blade 70. The blade 70 can be formed by crystal anisotropic etching using such a mask pattern 80. In the blade 70, a tip 72 is one in which a <011> plane is formed on the two sides of the blade center line. The angle formed by the <011> surface and the back surface of the blade is 45°, and the angle formed by the line (center line) in which the two <011> planes intersect and the back surface of the blade is 55°. An edge 71 that extends rectilinearly is disposed so as to slope in relation to the center line of the blade from the tip 72 toward the rear section side. The edge 71 is a <122> plane and slopes 48° with respect to the back surface of the blade. The rear section side margin 73 of the blade 70 is a <111> plane that slopes 55° with respect to the back surface of the blade, and the rear corner section 74 is a <101> plane that slopes 45° with respect to the back surface of the blade. The rear end margin 75 of the blade is a <111> plane that slopes 55° with respect to the back surface of the blade.

FIG. 16(a) is top view showing a mask pattern 81 of the rim section when a single-crystal silicon having an orientation of <100> is used and a rimmed blade is formed, and FIG. 16(b) is a cross-sectional view along the line A-A of FIG. 16(a) and is a view that shows the result of etching the single-crystal silicon using the mask pattern 81. The mask pattern 81 is composed of a rim section 82 that surrounds the mask pattern 80 for a blade, and a rib section that extends from the rim section 82 toward the mask pattern 83 for a blade. An inclined surface (<111> plane) having an inclined angle 55° is formed, in the manner shown in FIG. 16(b), from the mask patterns 80 and 81.

FIG. 17 is a diagram showing a rimmed blade formed by crystal anisotropic etching using the mask pattern 80 and 81. FIG. 17(a) is a perspective view, FIG. 17(b) is an enlarged top view of the connection parts of the blade 70 and rib 92, FIG. 17(c) is a top view, and FIG. 17(d) is a perspective view as seen from the rear of the blade. The blade 70 has an edge 71 and a tip 72 that have inclined surfaces that slope at sharp angles formed, as shown in FIG. 14.

The angle of the distal end of the mask pattern is suitably set in the same manner as described above for the case in which the <100> wafer is used, whereby various higher-order planes can be formed by crystal anisotropic etching. The planes <122> and <011> described above are merely examples.

### Industrial Applicability

The present invention is preferable for a surgical knife used in ophthalmology or surgery.

## Claims

1. A surgical knife comprising:
a blade in which a single-crystal silicon wafer in which the orientation of a polished plane is <110> or <100> is subjected to crystal anisotropic etching, whereby an inclined plane sloped in relation to said polished surface is formed and an edge is formed between said inclined plane and said polishing surface of the wafer.

2. The surgical knife according to claim 1, wherein
the orientation of said polished surface is <110>; and
the inclined plane constituting said edge includes a higher-order plane formed by crystal anisotropic etching.

3. The surgical knife according to claim 1, wherein
the orientation of said polished surface is <100>; and
the inclined plane constituting said edge includes a higher-order plane formed by crystal anisotropic etching.

4. A blade for a surgical knife, comprising:
a blade in which a single-crystal silicon wafer in which the orientation of a polished surface is <110> or <100> is subjected to crystal anisotropic etching, whereby an inclined surface that slopes in relation to said polished surface is formed and an edge is formed between said inclined plane and said polished surface of wafer;
a rim disposed in a position that is set at a distance from the blade at the periphery of the blade; and
a rib for connecting said rim and the section of said blade in which said edge is not formed.

5. A method of manufacturing the blade for a surgical knife according to any of claims 1 to 4, comprising:
forming a mask pattern that includes a dummy section for inspection in addition to a product blade on said single-crystal silicon wafer; and
etching said silicon wafer via crystal anisotropic etching using said mask pattern as a mask, whereby said surgical knife is manufactured.

6. A handle for a surgical knife for separating the blade for a surgical knife according to claim 4 from said rib and forming a surgical knife in which the blade is mounted at a distal end, comprising:
a handle main body;
an open/close section which is disposed on the front end of the handle main body and in which a rear end section is rotatably supported on said handle main body so that a front end section opens and closes; and
a projection which is disposed on a side margin of the open/close section and which disconnects said rib when said open/close section is closed.
